# EUROPEAN PATENT APPLICATION

(11) **EP 4 442 682 A1**
(43) Date of publication of application: **09.10.2024**
(21) Application number: 24168297.0
(22) Date of filing: 03.04.2024
(51) Int. Cl.: C07D 251/60, F28D 7/00, F28D 7/10, F28D 20/00

(54) **AMMONIA HEATING GROUP FOR MELAMINE PRODUCTION PLANTS AND MELAMINE PRODUCTION PLANT USING THE SAME**

(30) Priority: 04.04.2023 IT 202300006591
(71) Applicant: Proman AG, 8832 Wollerau Schwyz (CH)
(72) Inventor: DE AMICIS, Alberto, I-20097 San Donato Milanese (MI) (IT); DI RUOCCO, Giuseppe, I-23900 Lecco (IT); SANTUCCI, Roberto, I - 21050 Gorla Maggiore (VA) (IT)
(74) Representative: De Gregori, Antonella

(57) **Abstract**

The present invention relates to a heating group for melamine production plants and to a melamine production plant using said group. The heating group (10) for melamine plants comprises a molten salt circuit (11) for supplying the reaction heat to a melamine plant (20) and a superheated ammonia supply unit (12) to the plant (20), the unit (12) comprising at least an evaporation exchanger (17) and a superheating exchanger (30), said unit being characterized in that the superheating exchanger (30) comprises at least one separate heat source (32) with respect to the molten salt circuit (11), said separate heat source (32) being superheated steam, said superheating exchanger (30) being a jacketed tube composed of two concentric tubes wherein the inner tube (31) is suitable for being traversed by a flow of ammonia to be superheated and the sealed outer tube or jacket (33) is suitable for being traversed by superheated steam, said inner tube (31) having an internal diameter which ranges from 20 mm to 85 mm, corresponding to lengths of the jacketed tube (30) ranging from 30 to 20 m respectively.

## Description

The present invention relates to an ammonia heating group for melamine production plants and a melamine production plant using said group.

In particular, the present invention relates to an ammonia heating group, in a plant for the production of melamine using a circuit of molten salts, which advantageously has a low risk of the formation of caustic soda and, consequently, a plant for the production of melamine wherein the SCC (stress corrosion cracking) phenomenon due to the presence of caustic soda in the molten salts is minimized or, preferably, eliminated.

Within the context of the present description, the abbreviation "SCC" indicates a corrosion process which evolves by forming intergranular or transgranular cracks which lead to leakages through the specific wall or to the complete collapse of a structure.

Within the context of a melamine production plant, the SCC can, for example, result in the collapsing of one or more tubes of the tube bundle of the synthesis reactor or of the part of the heat exchanger conceived for superheating the ammonia of the relative group.

In processes for the production of melamine, both at high and low pressure, a certain quantity of ammonia must be fed at high temperature into the reaction section, or into other areas of the process, in order to avoid local cooling which can lead to solidification of the melamine (which takes place at 354°C).

The ammonia is preferably fed at the reaction temperature approximately equal to 380°C. This reaction temperature is much higher than the critical temperature of ammonia (132.4°C), ammonia which is at the reaction temperature is therefore normally defined as superheated ammonia.

In melamine production processes, in particular in processes operating without a catalyst and therefore at high pressure, the ammonia is compressed to the desired pressure by means of pumps. For this purpose, reciprocating pumps are normally used, such as for example piston or diaphragm pumps, fed with liquid ammonia.

This ammonia is consequently brought to the desired temperature through various steps including a first step for increasing the temperature up to the boiling point in equilibrium with the pressure, a second step for boiling up to the complete passage of the ammonia from the liquid state to the gaseous state, and a third step for raising the temperature of the gaseous ammonia to the reaction temperature.

The first two steps for heating the ammonia are preferably effected through the use of heat exchangers, using, as heating fluid, low-, medium-, or high-pressure steam available in industrial complexes. Steam at 5-6 bar can be used, for example, for heating up to the boiling point and complete evaporation. The same steam can also be used for raising the temperature of the ammonia up to about 150°C, a temperature at which all ammonia is certainly in the gaseous phase.

It is therefore possible to continue raising the temperature of the ammonia by means of heat exchangers using, as heating fluid, medium- or high-pressure steam up to as much as possible, depending on the steam available in the industrial complex in which it is operating.

According to the state of the art prior to the present invention, for the third step for heating the ammonia, it is not possible to use steam as the temperature to be reached, i.e. approximately 380°C, is higher than the critical temperature of the water equal to 373.936°C, corresponding to a pressure of 220.58 bar.

According to the state of the art, therefore, in order to carry out this third step for heating the ammonia, electric superheaters are known to be used, or other heating fluids already present in melamine production plants and used for supplying the reaction heat and/or for keeping the equipment and/or pipes at a temperature higher than the solidification temperature of the melamine, i.e. at a temperature higher than 354°C, preferably at temperatures equal to or higher than 380°C.

In currently known melamine production plants, two fluids are normally available at temperatures suitable for the purpose, i.e. diathermic oil, for example Dowtherm, and molten salts, both managed and operated by means of two separate closed-loop circuits, each comprising a heating furnace, at least one circulation pump, a tank, the relative pipes, valves and anything else necessary for the management of the system.

Diathermic oil has a maximum temperature of continuous use of 380°C. Consequently, in order to avoid a rapid decomposition by cracking, this fluid is normally used for maintaining the temperature of fluids which are already at the reaction temperature. In other words it is used for preventing cooling of the melamine circulating in the pipes.

This maximum temperature of continuous use equal to 380°C, in fact, does not allow there to be a useful temperature difference for managing a heat exchanger for heating the ammonia which must be specifically heated to a temperature of 380°C.

The only fluid available and which can actually be used for superheating the ammonia therefore remains the mixture of molten salts which, moreover, constitute the heating fluid which also supplies the reaction heat.

As is known, molten salts consist of a mixture of sodium and potassium nitrites and nitrates kept continuously at temperatures above 380°C. This type of mixture is strongly oxidizing, so that in order to avoid contact with oxygen and atmospheric humidity, the molten salt circuit is kept under a positive nitrogen pressure at approximately 0.5 bar.

For the superheating of ammonia it is therefore known to use a heat exchanger made of a material which resists ammonia at 380°C such as an alloy with a high nickel content (high nickel alloy) of which an example is the alloy Hastelloy C276.

This type of material is also typically used for producing reactors for the production of melamine used in plants that use molten salts as heating fluid.

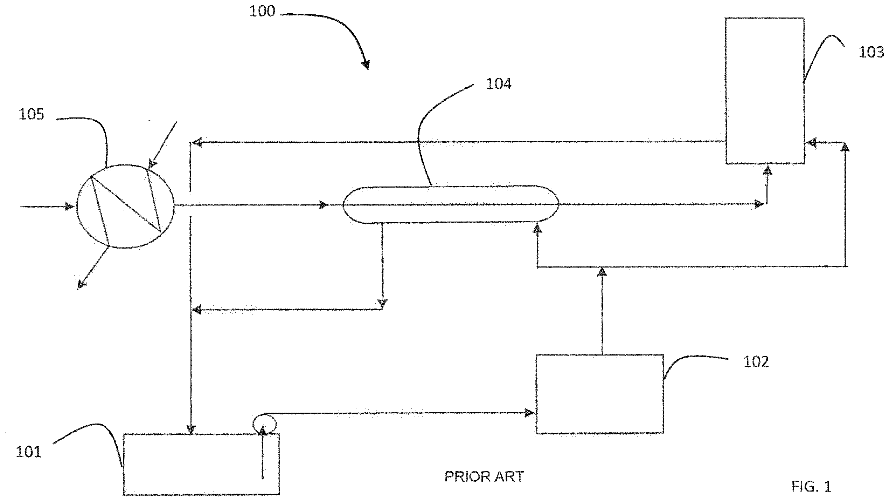

As shown in Figure 1, in a plant according to the state of the art, the molten salts are generally collected from a tank 101 and sent to a heating furnace 102 by means of a circulation pump. From the circulation pump, the molten salts are sent to the reactor 103 for the production of melamine and to the part of the heat exchanger conceived for superheating the ammonia, already evaporated and heated in the exchanger 105, also called superheating exchanger or, briefly, superheater 104 to then return, from both the reactor and from the superheater, to the tank 101 of the circuit.

The molten salts are then continuously stirred with no variation in the composition and temperature between the quantity used as the heating fluid of the reactor 103 and the quantity used as the heating fluid of the superheater 104.

In a heat exchanger in which the fluid to be heated is in the gaseous phase, this must circulate at a rate which is such as to generate a turbulent motion in order to have acceptable heat-transfer-coefficient values. Furthermore, residual drops of liquid may be present in a gas formed from an evaporated liquid, for example in the case of malfunctioning of the evaporation phase. There is therefore the risk of erosion/corrosion phenomena due to the presence of residual droplets moved at high speed by the turbulent motion necessary for obtaining a good heat exchange. These erosion/corrosion phenomena can cause perforation of the wall of the tube in which the fluid to be heated flows, putting it in communication with the area outside it and thus causing a leakage in the heat exchanger.

More specifically, a heat exchanger may be subject to internal leakages (i.e. when the two fluids of the exchanger (to be heated and heating) enter into reciprocal communication) and/or external leakages (i.e. when a fluid of the exchanger enters into communication with the atmosphere) according to the type of mechanical construction with which it is made. In the case of internal leakage, there is a mixing of the two fluids.

In a heat exchanger used for feeding superheated ammonia 104,105, the part most at risk is the area of the heat exchanger in which the ammonia reaches evaporation temperature. In this area, for a certain distance, a mixed phase of steam and liquid is present. This critical area of the heat exchanger is typically included in the part of the heat exchanger intended for the evaporation of the ammonia, also called evaporation exchanger (or evaporator for short) 105.

Due to anomalies, such as, for example, the malfunctioning of steam traps, the lowering or lack of steam pressure, the lack of adequate thermal insulation, etc., it is possible however, that drops of ammonia in liquid form be present also in correspondence with the superheating exchanger 104 which, as already explained, uses molten salts as heat source.

As in melamine production plants the ammonia is at a higher pressure than that of the molten salts, in the event of an internal leakage in the heat exchanger in correspondence with the superheater, ammonia enters the molten salt circuit with an immediate consequent reaction between the two products.

Due to their natural slow transformation, a certain amount of sodium oxide is always present in the molten salt mixture. Furthermore, the reaction of ammonia with molten salts leads to the formation of water, as molten salts (mixture of nitrites and nitrates) are an oxidizing compound. Consequently, in the case of contact between ammonia and molten salts, there is the formation of caustic soda due to the reaction between sodium oxide and water.

The quantity of ammonia that enters the molten salt circuit is directly proportional to the passage section.

If a large quantity of ammonia enters the molten salt circuit, a strong exothermic reaction takes place and the pressure and temperature rise in the molten salt circuit. This critical situation can be immediately detected, allowing the production plant to be promptly stopped for maintenance. In particular the molten salts must be replaced, as they will be polluted by the presence of ammonia and degraded due to the reaction with the ammonia which alters their composition.

In the case of a minimum entry of ammonia into the molten salt circuit, this anomaly is not immediately detectable as no significant exothermic reaction takes place, so the production plant continues to operate normally. The molten salts coming out of the ammonia superheater and that have reacted with ammonia to form water and, consequently, caustic soda, therefore return to the tank, mixing with the rest of the molten salts, then re-entering in circulation both in the superheater and, in greater quantities, in the reactor for the production of melamine.

The presence of molten salts containing caustic soda in a plant operating at a high operating temperature and in which the equipment is subjected to high mechanical stress (in particular the reactor and the superheater) determines the precondition for the formation of SCC phenomena, in particular in the presence of materials subject to said phenomena such as alloys with a high nickel content of which the reactor and the superheater are made. Even modest quantities of caustic soda present in the molten salts can therefore cause SCC phenomena in this equipment.

In particular, in relation to the reactor, this can lead to the perforation of one or more tubes of the tube bundle of the same with the consequent entry of melamine and ammonia into the molten salt circuit.

Furthermore, melamine, containing carbon, is subject to combustion in the presence of a strongly oxidizing substance such as the mixture of nitrites and nitrates of which the molten salts are formed. The entry of melamine into the molten salt circuit therefore creates the risk of a strong increase in pressure and temperature which can lead to an actual explosion in the circuit.

In order to reduce, or preferably completely eliminate, the risk of the creation of caustic soda in the molten salt circuit, minimize SCC (stress corrosion cracking) wear phenomena and prevent the situation in which melamine and/or ammonia can enter the molten salt circuit of a melamine production plant, the state of the art also provides, as an alternative to the use of molten salt exchangers, the use of heat exchangers which use electric current as heating means.

The ammonia heating group or electric superheater can use, as heating means, both direct heating by means of electric resistances and by connecting an electric energy source to the pipe in which the ammonia to be heated passes. In this specific case the pipe acts as an electrical conductor and is heated by means of the joule effect.

The use of the electric heating group, however, although preferable to the molten salt exchanger, has a high cost both in terms of energy and equipment as it is composed of various elements necessary for its safe functioning.

In order to overcome the drawbacks described above and inherent in known systems, the applicant has therefore found an alternative solution that allows the highest safety standards to be met, reducing the ecological impact as much as possible..

The present invention therefore relates to a heating group for melamine production plants comprising a molten salt circuit for supplying the reaction heat to a reactor of a melamine production plant and a unit for feeding superheated ammonia to the plant, comprising at least one evaporation exchanger and one superheating exchanger, said unit being characterized in that
- the superheating exchanger comprises at least one heating source separate from the molten salt circuit, this separate heating source being superheated steam,
- this superheating exchanger being a jacketed tube composed of two concentric tubes wherein the inner tube is suitable for being traversed by a flow of ammonia to be superheated and the sealed outer tube or jacket is suitable for being traversed by superheated steam and
- said inner tube having an internal diameter which varies from 20 mm to 85 mm, corresponding to lengths of the jacketed tube ranging respectively from 30 m to 20 m.

The superheating exchanger is preferably a jacketed tube wherein the inner tube has an internal diameter ranging from 21.6 mm to 55 mm, which corresponds to a preferred length of the jacketed tube ranging from 27 m to 23 m. Even more preferably, the inner tube has an internal diameter ranging from 24.6 to 50.8 mm, which corresponds to a length of the jacketed tube ranging from 25 m to 22 m.

This solution, surprisingly and contrary to the existing preconception, enables the use of superheated steam, already available in factories in which melamine production plants are located, where it is normally used as the driving force of the turbines, or that can be produced by recovering heat from the furnaces.

Keeping the superheated ammonia supply unit separate from the molten salt circuit, in fact, ensures that, even in the event of erosion/corrosion phenomena of the superheater used in this unit, the ammonia treated in the superheater cannot enter the circuit of molten salts. To this end, the heating group for a melamine production plant provides that the ammonia superheater only uses different heat sources with respect to the molten salt circuit. In this way, there is no contact between the superheater and the molten salt circuit and it is thus guaranteed that no possible ammonia leakages in correspondence with the superheater can come into contact with the molten salts.

As previously observed, in the superheater, the ammonia must be heated to a temperature of 380°C, the heating fluid must therefore necessarily be at a higher temperature in order to maintain a temperature difference which is such as to guarantee the passage of heat from the heating fluid to the fluid to be heated i.e. over 380°C. Superheated steam at temperatures higher than the critical temperature of water (equal to 373.936°C) has never been considered as being usable as a heating means in the ammonia superheater, as the quantity of usable heat, above the condensing temperature, is reduced to sensitive heat only. The use of sensitive heat alone would therefore entail the need for providing for enormous exchange surfaces compared to the use of the same quantity of heat exchanged under condensing steam conditions.

In order to overcome this preconception of the state of the art, the applicant has effected a series of process optimizations aimed at reducing the quantity of ammonia to be heated in order to also reduce as much as possible the amount of heat necessary for said heating and has surprisingly found that, using superheated steam at temperatures above 450°C and by suitably sizing the superheating exchanger for maximizing the heat exchange, the aim of superheating the ammonia is achieved by using steam and with a heat exchanger having a limited size and entirely acceptable; in particular a simple jacketed tube which satisfies the parameters claimed is used for the purpose.

The solution according to the present invention makes it possible, for example, to use superheated steam, normally present in the case of the internal production of electric energy by means of steam turbines, using a minimum quantity and thus not altering the requirements of the turbines; in this way it is possible to superheat the quantity of ammonia necessary for the production of the melamine plant, at the same time maintaining the superheating and pressure characteristics necessary for allowing the steam to still be used in the production of electric energy. In other words, the superheating temperature of the steam must remain such as to guarantee the non-formation of condensate at the operating pressure in the turbine and the pressure should remain such as to be sufficient for its supply at the inlet.

In conclusion, therefore, the solution according to the present invention allows a particularly important result to be obtained, i.e. to superheat the quantity of ammonia required for the melamine production plant with the superheated steam already present in the plant, at the same time continuing to use this superheated steam in turbines for the production of electric energy.

In order to demonstrate the efficiency in the superheating of ammonia by means of the superheating unit according to the present invention, some tests were carried out which allowed it to be verified how the optimization of the sizing of the heat exchanger surprisingly allowed the characteristics of the steam for its use in turbines to be maintained.

The solution according to the present invention in fact allows, in a particularly advantageous way, this superheated steam to be used for the superheating of ammonia, without reducing the availability of the steam to the turbine so as not to reduce the production of electric energy.

### Test 1: jacketed tube-type exchanger:

| | |
|---|---|
| steam flow-rate | 2,100 kg/h |
| inlet temperature of steam | 535°C |
| outlet temperature of steam | 372°C |
| inlet pressure of steam | 94.9 bar abs |
| outlet pressure of steam | 94.7 bar abs |
| dew point of steam | 307°C (at outlet pressure) |
| ammonia flow-rate | 1155 kh/h |
| inlet temperature of ammonia | 160°C |
| outlet temperature of ammonia | 380°C |
| internal diameter of ammonia tube | 81 mm |
| length of the ammonia tube | 24.3 m. |

The following tests 2, 3, 4 (according to the invention) and 5 (comparative test) were carried out maintaining the same initial feeding conditions of steam and ammonia (with variations in the second decimal of the steam outlet conditions which do not affect the assumptions of steam re-use), but varying the internal diameter of the inner tube of the jacketed tube in order to optimize the dimensions of the exchanger, as the variation of the internal diameter involves a variation in the exchange surface, the rate of the fluid and the residence time.

### Test 2:

| | |
|---|---|
| internal diameter of the ammonia tube | 50.8 mm |
| length of the ammonia tube | 22m |

### Test 3:

| | |
|---|---|
| internal diameter of the ammonia tube | 24.6 mm |
| length of the ammonia tube | 25 m |

### Test 4:

| | |
|---|---|
| internal diameter of the ammonia tube | 21.6 mm |
| length of the ammonia tube | 27 m |

### Test 5 (comparative):

| | |
|---|---|
| internal diameter of the ammonia tube | 17.4 mm |
| length of the ammonia tube | 37 m |

The above tests allowed it to be established that, for the ammonia flow-rate considered, which corresponds to the quantity of ammonia necessary for the functioning of a melamine synthesis plant, the best heat-exchange conditions are obtained with a jacketed-tube superheating exchanger wherein the inner tube has an internal diameter ranging from 24.6 to 50.8 mm, which corresponds to a length of the jacketed tube that can vary from 25 to 22 m, values which, contrary to what has so far been assumed by the state of the art, allow the use of superheated steam by exploiting only the sensitive heat without however precluding its subsequent use for powering the turbines.

Furthermore, this combination of internal diameter/length, within which the above tests 2-4 fall, also allows the costs of the equipment to be optimized, as the use of a larger internal diameter for the same length of tube is less efficient from the point of view of the overall cost of the equipment (increasing the thickness of the tube itself) and of the cost of the process which, at the same flow-rates and temperatures, thanks to the use of superheated steam, allows the use of a jacketed tube exchanger with much lower costs with respect to the electric superheater.

The use of this type of steam in fact results in zero energy costs.

The above is also made possible by the continuous optimization of the consumption of ammonia required in the reaction section, whose reduced quantity makes the use of superheated steam economical and safe.

The solution according to the present invention therefore allows the ammonia necessary for the melamine synthesis plant to be superheated using superheated steam both in the case in which it is already present inside the plant as superheated steam or by superheating steam already present in the plant by recovering heat from the fumes of the furnaces present in the melamine plant, for example by recovering the heat from the fumes of the furnaces used for heating the reactor.

This solution is therefore particularly advantageous as it allows the use of heat sources already present in the plant, no further CO₂ is produced and it allows a simple device to be used as a superheating exchanger for the ammonia without additional service machines present in electric superheaters (transformers, fans, pressurization with nitrogen, etc.), it has lower costs with respect to the use of electric superheaters and eliminates the risks associated with the use of molten salt superheaters previously indicated.

In accordance with a second aspect thereof, the invention relates to a plant for the production of melamine comprising at least one reactor for the production of melamine from urea and a heating group for supplying the reaction heat and superheated ammonia to at least the reactor, the heating group comprising a molten salt circuit and a superheated ammonia supply unit in turn comprising at least one evaporation exchanger and a superheating exchanger, said unit being characterized in that
- the superheating exchanger comprises at least one heat source separate from the molten salt circuit, this separate heat source being superheated steam
- said superheating exchanger being a jacketed tube composed of two concentric tubes wherein the inner tube is suitable for being traversed by a flow of ammonia to be superheated and the sealed outer tube or jacket is suitable for being traversed by superheated steam and
- said inner tube having an internal diameter which ranges from 20 mm to 85 mm, corresponding to lengths of the jacketed tube which range from 30 to 20 m respectively.

The plant for the production of melamine according to the invention advantageously achieves the technical effects described above in relation to the heating group for the melamine production plant.

The present invention in at least one of the above aspects may have at least one of the following preferred features; the latter can in particular be combined with each other as desired in order to satisfy specific application requirements.

The superheating exchanger is a jacketed tube composed of two concentric tubes wherein the inner tube is suitable for being traversed by a flow of ammonia to be superheated and the sealed outer tube or jacket is suitable for being traversed by superheated steam: it therefore comprises two concentric tubes in which ammonia (in the inner tube) and superheated steam (in the sealed outer tube or jacket or casing) flow, wherein the exchanger is then thermally coupled with a heat source independent of the molten salt circuit or electric energy.

The heat source of the superheating exchanger is preferably superheated steam already present in the melamine synthesis plant or alternatively it is steam already present in the melamine plant which is specifically superheated.

The use of a jacketed tube as superheating exchanger offers the advantage of obtaining a uniform distribution of heat, avoiding particularly dangerous local superheating in the case of ammonia treatment.

The inner tube in which the ammonia flows is preferably made of an ammonia-resistant material.

The ammonia-resistant material is more preferably an alloy with a high nickel content.

In this way it is advantageously ensured that the superheating exchanger is made of a material suitable for withstanding the operating conditions of the plant.

The evaporation exchanger preferably uses steam available in the melamine production plant as a heating fluid.

The use of a heating fluid already present in the system advantageously allows savings to be obtained in terms of costs and complexity of the system as a whole.

The molten salt circuit preferably comprises a circulation pump, a molten salt tank and a heating furnace, wherein the circulation pump creates at least a flow of molten salts from the tank to the heating furnace, from the heating furnace to the reactor and from the reactor to the tank without passing through the superheating exchanger.

It is thus advantageously ensured that the molten salts are not sent towards the superheating exchanger, thus avoiding any contact of the molten salts with the pipe in which ammonia flows inside the superheater.

Further characteristics and advantages of the present invention will become more evident from the following detailed description of some of its preferred embodiments, prepared with reference to the attached drawings.

The different features in the individual configurations can be combined with each other as desired according to the previous description, if the advantages specifically resulting from a particular combination are to be used.
In said drawings,
- figure 1 is a block diagram of a heating group for melamine production plants of the state of the art;
- figure 2 is a block diagram of a heating group for melamine production plants according to an embodiment of the present invention.

In the following description, for the illustration of the figures, identical reference numbers are used for indicating construction elements with the same function. Furthermore, for clarity of illustration, some reference numbers may not be repeated in all the figures.

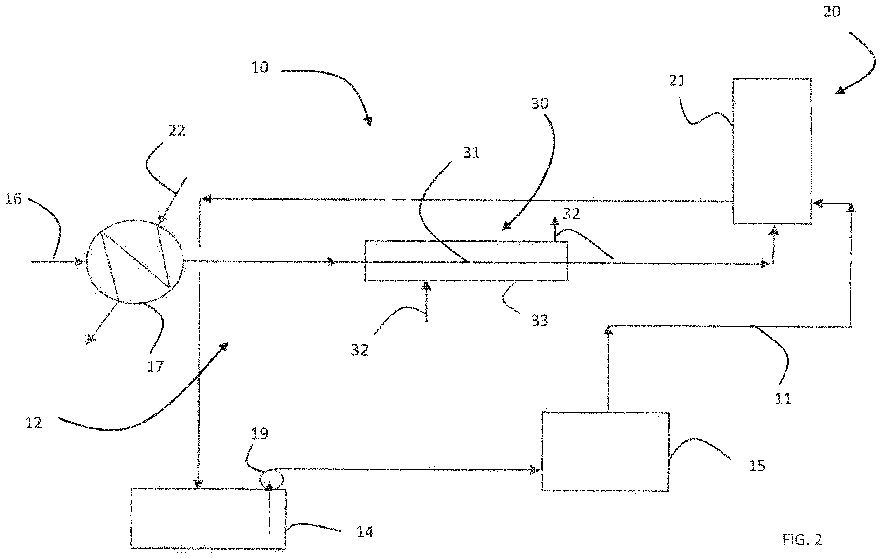

With reference to figure 2, a heating group for melamine production plants is shown, indicated as a whole with 10.

The heating group 10 comprises a circuit of molten salts 11 for supplying the reaction temperature to a melamine production plant 20 of which only the reactor 21 is shown in simplified terms in figure 2. In particular, the reaction temperature of a melamine production plant is substantially equal to 380°C.

The circuit of molten salts 11 comprises a circulation pump 19, a molten salt tank 14 and a heating furnace 15. The circulation pump 19 creates a flow of molten salts from the tank 14 to the heating furnace 15. The molten salts are sent from the heating furnace 15 only to the reactor 21 of the melamine production plant 20 again through the action of the circulation pump 19 present in the circuit of molten salts 11. Finally, the molten salts return from the reactor 21 to the tank 14.

The heating group 10 additionally comprises a unit 12 for feeding superheated ammonia to the melamine production plant 20, for example, and not only to the reactor 21 of said plant.

In particular, the feeding unit of superheated ammonia 12 receives liquid ammonia 16 at the inlet and brings it to a temperature preferably equal to the reaction temperature.

The feeding unit of superheated ammonia 12 comprises an evaporation exchanger 17 suitable for heating liquid ammonia 16 until it substantially passes completely from the liquid state to the gaseous state and preferably at a temperature higher than about 150°C. The evaporation exchanger preferably uses as heat source a heating fluid already available in the melamine production plant 20 and in particular steam 22.

The superheated ammonia supply unit 12 also comprises a superheating exchanger 30 suitable for bringing the ammonia leaving the evaporation exchanger 17 to the reaction temperature.

The superheating exchanger 30 is a jacketed tube consisting of two concentric tubes wherein the inner tube 31 having any shape, for example straight as shown in figure 2, is made of a material suitable for resisting ammonia under the operating conditions, such as an alloy with a high nickel content, through which the ammonia passes.

The superheating exchanger 30 comprises, in addition to the inner tube 31 in which the ammonia passes, an outer concentric and sealed tube 33, in which a heating means passes which, according to the present invention, is not molten salts but, on the contrary, a heat source 32, independent of the molten salt circuit 11, consisting of superheated steam.

The superheating exchanger 30 is connected upstream, with respect to the ammonia flow, to the evaporation exchanger 17 and downstream to the reactor 21, for feeding superheated ammonia leaving the superheater 30 to the reactor 21.

The heat source 32 of the superheating exchanger 30 is superheated steam.

The superheating exchanger 30 preferably additionally comprises temperature sensors (not shown in the figures) positioned so as to be able to detect the temperature of the ammonia and of the incoming and outgoing steam by regulating the flow-rate of the steam in order to ensure the desired temperature of the ammonia (380°C) without the temperature of the outgoing steam dropping excessively, approaching the condensing temperature at operating pressure.

According to another alternative embodiment (not shown in the figures) the heat source 32 is a source of superheated steam by heat recovery from the fumes of the furnace used for heating the reactor.

From the description provided, the characteristics of the heating group for melamine production plants and the relative melamine production plant object of the present invention are evident, as also the relative advantages.

From the embodiments described above, further variants are possible, without departing from the teaching of the invention.

Finally, it is evident that a heating group for melamine production plants and a melamine production plant thus conceived can undergo numerous modifications and variations, all falling within the scope of the invention; all the details, moreover, can be replaced by technically equivalent elements. In practice, the materials used, as also the dimensions, can vary according to technical requirements.

## Claims

1. A heating group (10) for melamine production plants comprising a circuit of molten salts (11) for supplying the reaction heat to a melamine production plant (20) and a superheated ammonia supply unit (12) to said plant (20), said unit (12) comprising at least one evaporation exchanger (17) and a superheating exchanger (30), said unit being **characterized in that**
- the superheating exchanger (30) comprises at least one separate heat source (32) with respect to the molten salt circuit (11), said separate heat source (32) being superheated steam,
- said superheating exchanger (30) being a jacketed tube composed of two concentric tubes wherein the inner tube (31) is suitable for being traversed by a flow of ammonia to be overheated and the sealed outer tube or jacket (33) is suitable for being traversed by superheated steam and
- said internal pipe (31) having an internal diameter which ranges from 20 mm to 85 mm, corresponding to lengths of the jacketed tube (30) ranging from 30 to 20 m respectively.

2. The heating group (10) for melamine production plants according to claim 1, **characterized in that** the superheating exchanger (30) is a jacketed tube wherein the inner tube (31) has an internal diameter ranging from 21.6 mm to 55 mm, corresponding to a length of the jacketed tube (30) ranging from 27 m to 23 m, more preferably the inner tube (31) has an internal diameter which ranges from 24.6 to 50.8 mm, corresponding to a length of the jacketed tube (30) which ranges from 25 m to 22 m.

3. The heating group (10) for melamine production plants according to claim 1 or 2, **characterized in that** the superheating exchanger (30) comprises two concentric tubes (31, 33) wherein ammonia flows in the inner tube (31) and superheated steam in the sealed outer tube or jacket or casing (33), wherein the exchanger (30) is then thermally coupled with a heat source (32) independent of the molten salt circuit (11).

4. The heating group (10) for melamine production plants according to any of claims 1 to 3, **characterized in that** said heat source (32) of the superheating exchanger (30) is superheated steam flowing in the outer tube (33 ) which constitutes the jacket of the inner tube (31) in which ammonia flows.

5. The heating group (10) for melamine production plants according to any of claims 3 to 4, **characterized in that** the inner tube (31) in which ammonia flows is made of an ammonia-resistant material, preferably an alloy with a high nickel content.

6. The heating group (10) for melamine production plants according to any of the previous claims, **characterized in that** the evaporation exchanger (17) uses steam (22) available in the melamine production plant (20) as heating fluid.

7. The heating group (10) for melamine production plants according to any of the previous claims, **characterized in that** said molten salt circuit (11) comprises a circulation pump (19), a molten salt tank (14) and a heating furnace (15), wherein the circulation pump (19) creates at least a flow of molten salts from the tank (14) to the heating furnace (15), from the heating furnace (15) to the reactor (21) and from the reactor (21) to the tank (14) without passing through said superheating exchanger (30).

8. A plant for the production of melamine (20) comprising at least one reactor (21) for the production of melamine from urea and a heating group (10) for supplying at least to said reactor (21), the reaction heat and superheated ammonia according to any of the previous claims.
